# EUROPEAN PATENT SPECIFICATION

(11) **EP 0 672 163 B1**
(45) Date of publication and mention of the grant of the patent: **26.02.1997**
(21) Application number: 93917333.2
(22) Date of filing: 28.07.1993
(51) Int. Cl.: C12P 19/04, C11D 3/22, C08B 37/00

(54) **Heteropolysaccharide 35-80 obtainable from Agrobacterium species ATCC 55341**
Aus Agrobacterium species ATCC 55341 erhaltenes Heteropolysaccharid 35-80
Hétéropolysaccharide 35-80 susceptible d'être obtenu à partir d'Agrobacterium species ATCC 55341

(30) Priority: 18.09.1992 US 947191
(43) Date of publication of application: 20.09.1995
(73) Proprietor: Cultor Ltd., 00240 Helsinki (FI)
(72) Inventor: DASINGER, Bruce, L., Niantic, CT 06357 (US)
(74) Representative: Patentanwälte Zellentin & Partner
(86) International application number: US9306969
(87) International publication number: WO9406927

(56) References cited:
- EP-A- 0 230 346
- EP-A- 0 351 303
- US-A- 4 269 939

## Description

This invention pertains to the field of microbial polysaccharides. In particular, these polysaccharides occur in the form of exocellular heteropolysaccharides which are high molecular weight, generally linear or branched, carbohydrates containing two or more different kinds of monosaccharides forming repeating units that are polymerized.

These heteropolysaccharides are widely used in a variety of applications, including industrial foods, well drilling, agricultural, paint, etc. Commercial demand for these heteropolysaccharides continues to increase.

One of the most widely used heteropolysaccharides is xanthan, or xanthan gum, which is produced during fermentation by bacteria of the genus Xanthomonas, typically Xanthomonas campestris. This xanthan heteropolysaccharide contains glucose, mannose and glucuronic acid. Various industrial uses of xanthan gum are known, see e.g. United States Patent Nos. 3,326,305 and 4,244,826.

Another widely useful class of heteropolysaccharides are the succinoglycans, a class of exocellular heteropolysaccharides produced by bacteria of the genera Alcaligenes, Agrobacterium and Pseudomonas. These succinoglycans contain galactose, glucose and variable proportions of acid residues such as pyruvate, succinate and acetate. Industrial uses for these succinogycans are also known, see eg., European Patent Office Application No. 040445, and United States Patent Nos. 4,339,239, 4,347,289 and 4,298,795.

In one embodiment, the present invention is directed to a heteropolysaccharide having the designation 35-80 and produced by growing Agrobacterium species ATCC 55341 in an aqueous medium by aerobic fermentation of an assimilable carbon source, said heteropolysaccharide containing the neutral sugars glucose and galactose in the approximate molar ratio of 7:1, said heteropolysaccharide being free of uronic acid derivatives.

In a preferred embodiment, the heteropolysaccharide is characterized by stability over a pH range of from about 2 to about 12.

Preferably, the heteropolysaccharide is stable to bases selected from the group consisting of sodium hydroxide and potassium hydroxide and caustic solutions of said bases.

In another preferred embodiment, the heteropolysaccharide is capable of forming gels by being cross-linked with a polyvalent metal cation. Preferred polyvalent metal cations capable of cross-linking the heteropolysaccharide are titanium (IV), aluminum (III) and chromium (III).

Preferred industrial applications of the heteropolysaccharide are as a thickener for acid and caustic cleaning solutions.

FIG. 1 illustrates the pH stability of heteropolysaccharide 35-80.

FIG. 2 illustrates the caustic stability of heteropolysaccharide 35-80.

The Agrobacterium species producing heteropolysaccharide 35-80 was isolated from a soil sample collected at El Coego, Honduras.

The heteropolysaccharide produced by Agrobacterium sp. ATCC 55341 is comprised principally of carbohydrate with about 5.9% substituent pyruvate groups, 8.4% succinate groups, and 0.32% acetate groups. The carbohydrate portion is comprised of glucose and galactose in the approximate ratio 7:1. This composition is characteristic of succinoglycans.

In order to determine the composition, purified polymer was obtained by diluting broth twenty-fold with distilled water, removing cells by centrifugation at 18,000 x g for 30 minutes, adding sodium chloride to 0.1% and precipitating polymer by addition of 3 volumes of isopropanol based on the volume of diluted polymer solution. The polymer precipitate was removed by filtration through a 149 µm (100 mesh) screen and washed by repulping in 100 milliliters of 70% isopropanol followed by filtration through a 149 µm (100 mesh) screen. Material was collected and dried by lyophilization to constant weight (usually 48 hours). Polymer was hydrolyzed with 2M trifluoroacetic acid (TFA) by dissolving ten milligrams of purified polymer in 2 milliliters distilled water, adding 2 milliliters of 4M TFA and heating the solution in a sealed glass ampoule for 4 hours at 120°C. TFA was removed by evaporation under vacuum at room temperature over sodium hydroxide overnight. Traces of TFA were removed by two cycles of dissolving the residue in distilled water and redrying under vacuum. Neutral sugars and uronic acid were tentatively identified by descending paper chromatography on Whatman® 3MM paper. The chromatogram was developed using a solvent system composed of butanol, glacial acetic acid, and water in the volumetric ratio of 6:4:3. Chromatograms were air dried and treated with a solution prepared by mixing 0.1 milliliters of a saturated aqueous solution of silver nitrate with 20 milliliters of acetone. Sugars and uronic acids react with silver nitrate to give gray to black spots depending on concentration. Spots were identified by chromatography of sugar and uronic acid standards. Locations of spots were consistent with a composition of glucose and galactose. There was no uronic acid spot present. Sugars in the hydrolysate were further identified and quantitated by gas-liquid chromatography of the alditol acetate derivatives [Hisamatsu, et al., Carbohydr. Res., 61, 89 (1978)]. A ratio of glucose:galactose in the approximate range of 7:1 was obtained. Organic acid substituents of polymer 35-80 were assayed after their removal by mild acid hydrolysis (2N sulfuric acid, 100°C, 60 minutes) according to the procedure of Hisamatsu, et al., 1978, Supra. Separation was accomplished by isocratic cation chromatography (Biorad Aminex HPX-87H) using a mobile phase of 0.013N sulfuric acid, with UV detection at 206 nm. In two separate analyses, pyruvate comprised 5.9%, succinate comprised about 8.4% and acetate comprised about 0.32%.

Polymer 35-80 is a viscosity building agent for aqueous media over a broad pH range of 0 to 12. This suggests its utility in thickening a variety of acidic and caustic media, especially caustic cleaners. Also, it forms strong gels with a number of multivalent metal cations including aluminum, chromium, and particularly titanium making it useful for applications in air fresheners, as a gelant for oil field application and for personal care products, such as deodorants.

### EXAMPLE 1

Fermentation Procedure for Producing Heteropolysaccharide 35-80.

### A. Culture Maintenance

Agrobacterium sp. ATCC 55341 grows well on the medium described in Table I. The same medium, with minor variations as specified, is used for fermenter seed preparation and final stage fermentation medium.

**TABLE I**

| Agar Plate Medium for Culture Maintenance and Culture Purification: | |
|---|---|
| Glucose | 3% |
| KH₂PO₄ | 0.01% |
| Yeast Extract | 0.025% |
| MgSO₄•7H₂O | 0.025% |
| NH₄NO₃ | 0.09% |
| CaCO₃ | 0.3% |
| Trace metal solution | 0.5 ml/liter medium |
| Agar | 1.8% |
| Trace Metal Stock Solution Composition | |
| Ingredient mg/l distilled water | |
| Boric acid | 285 |
| MnCl₂•4H₂O | 1800 |
| FeSO₄•7H₂O | 1360 |
| Sodium Tartrate | 2.098 |
| CuCl₂ | 26.9 |
| ZnCl₂ | 20.8 |
| CoCl₂•6H₂O | 74 |
| Na₂MoO₄•2H₂O | 25.2 |

The ingredients were dissolved or suspended in distilled water and sterilized by autoclaving for 30 minutes at 121°C. After cooling to 50°C, the agar medium was dispensed into Petri plates. Colonies of Agrobacterium sp. ATCC 55341 grown for 3 days at 30°C were white, raised, and mucoid.

### B. Seed Preparation

The medium was the same as that shown in Table I except that agar was omitted. Medium was prepared and dispensed (100 milliliters of medium into each 300 ml flask). Flasks were sterilized by autoclaving for 30 minutes at 121°C. After cooling, flasks were inoculated with a loopful of a 3 day old culture from an agar plate. Flasks were incubated at 300C with shaking at 200 rpm for 24 hours and 1 milliliter transferred to each new Seed Flask (second stage inoculum) of identical composition and volume)) which was incubated with shaking at 30°C for 48 hours.

### C. Fermentation Production Medium

The medium was the same as that shown in Table I except that agar was omitted and CaCO₃ was increased to 0.5%. The medium was prepared as described below and dispensed into a 15 liter New Brunswick fermenter. Ingredients for 10 liters of medium (omitting glucose) were prepared in 8.5 liters of distilled water. The fermenter contents were sterilized by autoclaving at 121°C for one hour. One liter of a 40% glucose solution was sterilized separately at 121°C for one hour and added aseptically to the fermenter. The fermenter was inoculated with 0.5 liter of second stage inoculum. The fermentation temperature was maintained at 30°C and the air rate was 3 liters/minute. The pH was maintained at 7.5 during fermentation by addition of sodium hydroxide using a pH controller. The initial agitation rate was 300 rpm, which was raised to 400 rpm and 500 rpm at 24 and 48 hours, respectively. The fermentation was terminated at 72-96 hours. Broth viscosity measured with a Brookfield model LVT viscometer using a #4 spindle at 6 rpm ranged from 25 to 35 Pa.s (25,000 to 35,000 centipoise). The fermenter broth was preserved by adding 3,000 ppm formaldehyde. The polymer concentration as determined by isopropanol precipitation of a preparation clarified to remove cells was 2%. The viscosity of a 2850 ppm solution of broth in 500 ppm NaCl was 0,410 Pa.s (410 centipoise) at 5.1 sec⁻¹ using a Fann Model 35A viscometer and a B-1 bob with a R-1 rotor.

### ATCC CHARACTERIZATION

Isolate identified as: Agrobacterium radiobacter, biovar I.

Morphology: Cells are gram-negative, aerobic, non-sporeforming, motile rods, peritrichously flagellated. Colonies are entire, smooth, glistening, translucent and cream colored.

| Physiology and Biochemistry: | | | |
|---|---|---|---|
| Gram positive | - | Casein hydrolysis | - |
| Gram negative | + | Starch hydrolysis | - |
| Gram variable | - | Gelatinase | - |
| Motile at 37°C | + | Tween® 20 hydrolysis | - |
| Motile at RT | + | Tween® 80 hydrolysis | - |
| Flagella peritrichous | + | Indole | - |
| Flagella lophotrichous | - | Simmons citrate growth | + |
| Flagella monotrichous | - | Urease | + |
| Flagella lateral | - | Nitrate to nitrite | + |
| 4°C growth | + | Nitrate reduction | + |
| 25°C growth | + | Nitrite to nitrogen gas | - |
| 30°C growth | + | Hydrogen sulfide (TSl) | - |
| 37°C growth | + | Lysine decarboxylase | - |
| 41°C growth | - | Arginine (Mollers) | - |
| Fluoroescein produced | - | Ornithine decarboxylase | - |
| Pyocyanine produced | - | Lecithinase | - |
| Diffusible orange | - | Phosphatase | - |
| Diffusible yellow | - | Catalase | + |
| Diffusible purple | - | Oxidase | + |
| Non-diffusible green | - | Growth on malonate as SCS | - |
| Other non-diff. pigments | - | dl-hydroxybutyrate growth | + |
| pH 6.0 growth | + | PHB accumulation | - |
| 3% NaCl growth | + | Growth on 0.05% centrimide | - |
| 6.5% NaCl growth | - | Testosterone deg. | - |
| MacConkey agar growth | + | Carrot tumorgenicity | - |
| Skim milk agar growth | + | Root hair formation | - |
| Aesculin hydrolysis | + | 3-Ketolactose from lactose | + |
| | | Deoxyribonuclease | - |

| Oxidative Fermentative Reactions in Hugh & Leifson's Medium: | |
|---|---|
| Acid from: | |
| L-arabinose | + |
| cellobiose | + |
| ethanol | + |
| D-fructose | + |
| D-glucose Aerobic | W |
| D-glucose Anaerobic | - |
| Alkaline pH in D-Glucose | - |
| glycerol | W |

| Acid from: | |
|---|---|
| i-inositol | W |
| lactose | W |
| maltose | W |
| D-mannitol | + |
| D-mannose | + |
| L-rhamnose | - |
| D-ribose | + |
| sucrose | + |
| trehalose | W |
| D-xylose | W |
| Control | K |
| | |
| + = acid | K = alkaline |
| - = no change | W = weak acid |

| Sole Carbon Sources in Stanier's Mineral Base: | | | |
|---|---|---|---|
| L-arabinose | + | D-xylose | + |
| cellobiose | + | adonitol | + |
| D-fructose | + | erythritol | - |
| D-glucose | + | glycerol | + |
| lactose | + | ethanol | W |
| maltose | + | geraniol | - |
| D-mannitol | + | i-inositol | + |
| L-rhamnose | + | sebacic acid | - |
| D-ribose | + | acetamide | - |
| D-sorbitol | + | acetate | + |
| sucrose | + | adipate | - |
| trehalose | + | benzoate | - |
| | | butyrate | - |
| citraconate | - | glycine | - |
| D-gluconate | + | L-histidine | + |
| M-hydroxybenzoate | - | DL-norleucine | - |
| 2-ketogluconate | + | L-proline | + |
| DL-lactate | + | D-tryptophan | - |
| L-malate | + | L-valine | - |
| pelargonate | - | DL-arginine | + |
| propionate | + | benzylamine | - |
| quinate | + | butylamine | - |
| succinate | + | putrescine | - |
| L-tartrate | - | mesoconate | - |
| valerate | - | DL-glycerate | + |
| B-alanine | + | L-tryptophan | - |
| D-A-alanine | + | Methanol | - |
| betaine | + | | |
| | | Control | - |

Phytopathogenicity tests were conducted on discs of fresh carrots. No tumors or hairy roots were formed. This is typical for Agrobacterium radiobacter, biovar 1.

### EXAMPLE 2

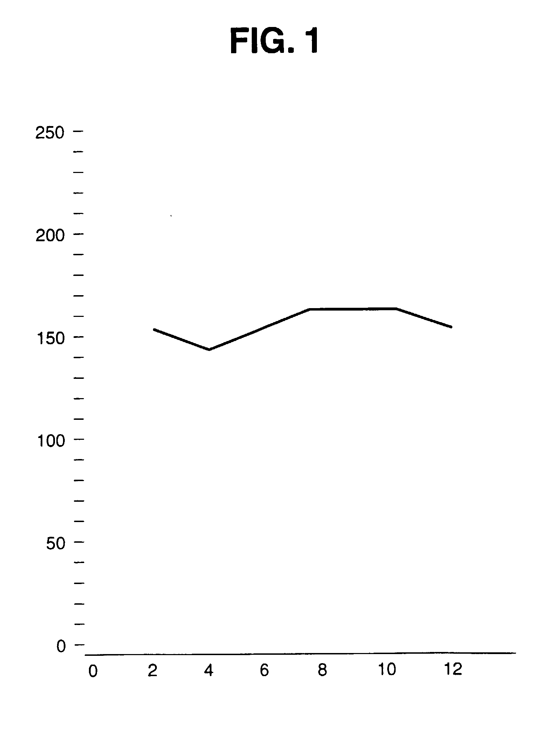

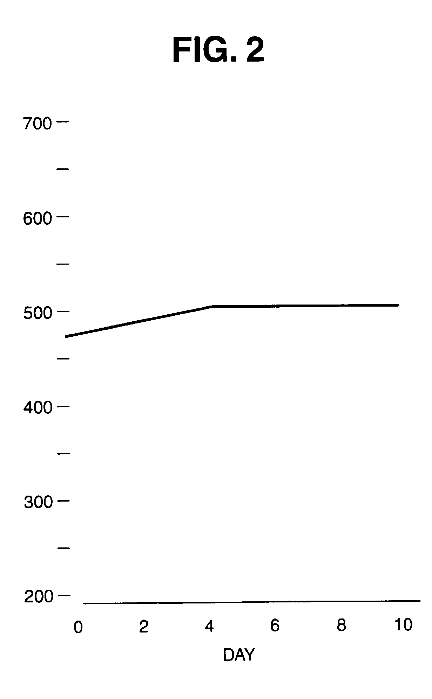

Biopolymer 35-80 exhibits a relatively flat viscosity profile over a very wide pH range of 2 to 12 (Figure 1). This suggests the suitability of biopolymer 35-80 for thickening a variety of acidic and caustic media. One particularly severe example showing excellent stability in 1% caustic (NaOH) is shown in Figure 2. In each of the above cases, solutions are prepared on a Waring^{R} blender by slow addition of biopolymer into a vortex (maintained by continual adjustment of blender voltage with a rheostat) of dilution water (containing the appropriate level of pH buffer or sodium hydroxide, respectively) followed by two minutes shear at 50 volts. The viscosities of the resulting solutions were measured on a Brookfield LVTD viscometer using the small sample adapter (SSA) and a #18 spindle at 6 rpm.

### EXAMPLE 3

Biopolymer 35-80 has also been shown to form excellent strong gels with a number of multivalent metal cation crosslinkers including aluminum, chromium, and titanium. Particularly good gels are made with titanium crosslinkers as is illustrated in Table 2. In each case, gels were formed by rapidly combining biopolymer 35-80 and crosslinker solutions.

The biopolymer solution was prepared by dissolving an appropriate quantity in 500 ppm Total Dissolved Solids (TDS) water (brine containing solution). A standard Waring^{R} blender dissolution method requires slow biopolymer addition into a dilution water vortex (maintained by continual adjustment of blender voltage with a rheostat) followed by 2 minute shear at 50 volts. Commercially prepared, assayed crosslinker solutions containing about 10% active cation were either used directly (e.g., Ti⁺⁴ gels) or diluted with chelator to control gelation rate and syneresis (e.g., 0.3 to 1 mole citrate per mole Al⁺³). Crosslinker was rapidly added (5 seconds with shearing at 70 volts) to the biopolymer solution and 40 gram aliquots were immediately poured into 60 ml (cc) Nalgene^{R} cups. After 24 and 48 hour setting times, gel strengths were measured on a modified Bloom gelometer. This test consisted of placing the gel sample on a tared digital balance, pressing the flat end of a standard diameter (1.3 cm) precision stainless steel piston into the center of the gel surface at a constant rate (2 mm/minute) and recording the gel's resistance to the applied force (as change in gel weight of the digital balance) until the gel surface broke. The modification to the standard Bloom gelometer is the use of a Sage Model 355 syringe pump to hydraulically drive the piston at a very carefully controlled rate and to generate the higher breaking pressures (200 g/cm²) required by these unusually strong gels.

**TABLE 2**

| GEL STRENGTHS FOR TI⁺⁴ CROSSLINKED BIOPOLYMER 35-80 Modified Bloom Gelometer, pH 4.0 | | |
|---|---|---|
| ppm Active Biopolymer 35-80 | ppm Ti⁺⁴ | Gel Strength g/cm² |
| | | (48 hours) |
| 5000 | 250 | 563 |
| 5000 | 500 | 403 |

## Claims

1. The heteropolysaccharide designated biopolymer 35-80 produced by the process of growing Agrobacterium species ATCC 55341 in an aqueous medium by aerobic fermentation of an assimilable carbon source, said heteropolysaccharide containing the neutral sugars glucose and galactose in the approximate molar ratio of 7:1, said heteropolysaccharide being free of uronic acid derivatives.

2. The heteropolysaccharide according to claim 1 characterized by solution viscosity stability over a pH range of from about 0 to about 12.

3. The heteropolysaccharide according to claim 1 further characterized by being stable to bases selected from the group consisting of sodium hydroxide and potassium hydroxide.

4. The polysaccharide according to claim 1 capable of forming gels by being crosslinked with polyvalent metal cations selected from the group consisting of Ti (IV), Al (III) or Cr (III).

5. A caustic cleaning solution having incorporated therein an effective thickening amount of biopolymer 35-80 as featured in claim 1.

6. An acidic cleaning solution having incorporated therein a thickening amount of biopolymer 35-80 as featured in claim 1.

7. A process for the preparation of a heteropolysaccharide designated biopolymer 35-80 containing the neutral sugars glucose and galactose in the approximate molar ratio of 7:1, said heteropolysaccharide being free of uronic acid derivatives, comprising growing Agrobacterium species ATCC 55341 in an aqueous medium by aerobic fermentation of an assimilable carbon source.

8. A microorganism having the identifying characteristics of Agrobacterium species ATCC 55341.

## Patentansprüche

1. Heteropolysaccharid, bezeichnet als Biopolymer 35-80, hergestellt durch das Verfahren Agrobacterium Spezies ATCC 55341 in einem wäßrigen Medium durch aerobe Fermentation einer assimilierbaren Kohlenstoffquelle zu züchten, wobei die Heteropolysaccharide die neutralen Zucker Glucose und Galactose in einem ungefähren molaren Verhältnis von 7:1 enthalten und die Heteropolysaccharide frei sind von Uronsäurederivaten.

2. Heteropolysaccharide gemäß Anspruch 1, **dadurch gekennzeichnet**, daß die Lösungsviskosität in einem pH-Bereich von ungefähr 0 bis ungefähr 12 stabil ist.

3. Heteropolysaccharide gemäß Anspruch 1, **dadurch gekennzeichnet**, daß sie stabil sind gegenüber Basen ausgewählt aus der Gruppe bestehend aus Natriumhydroxid und Kaliumhydroxid.

4. Polysaccharid gemäß Anspruch 1, welches in der Lage ist, Gele zu bilden durch Quervernetzung mit polyvalenten Metallkationen, ausgewählt aus der Gruppe bestehend aus Ti (IV), Al (III) oder Cr (III).

5. Eine alkalische Reinigungslösung, welche eine für das Verdicken ausreichende Menge des Biopolymers 35-80 gemäß Anspruch 1 enthält.

6. Eine saure Reinigungslösung, welche eine verdickende Menge des Biopolymers 35-80 gemäß Anspruch 1 enthält.

7. Ein Verfahren zur Herstellung eines Heteropolysaccharids bezeichnet als Biopolymer 35-80, enthaltend die neutralen Zucker Glucose und Galactose im ungefähren molaren Verhältnis von 7:1, wobei das genannte Heteropolysaccharid frei von Uronsäure-Derivaten ist, **dadurch gekennzeichnet**, daß das Agrobakterium. Spezies ATCC 55341, in einem wässrigen Medium unter aerober Fermentation einer assimilierbaren Kohlenstoff Quelle wächst.

8. Einen Mikroorganismus, der die das Agrobakterium ATCC 55431 kennzeichnenden Merkmale aufweist.

## Revendications

1. Hétéropolysaccharide désigné par l'appellation "biopolymer 35-80" obtenu par le procédé consistant à faire croître l'espèce Agrobacterium ATCC 55341 dans un milieu aqueux par fermentation aérobie d'une source de carbone assimilable, ledit hétéropolysaccharide contenant les sucres neutres de glucose et de galactose dans le rapport molaire approximatif de 7:1, ledit hétéropolysaccharide étant exempt de dérivés de l'acide uronique.

2. Hétéropolysaccharide selon la revendication 1, caractérisé par une stabilité de la viscosité en solution sur un domaine de pH d'environ 0 à environ 12.

3. Hétéropolysaccharide selon la revendication 1, caractérisé en outre par le fait qu'il est stable vis-à-vis de bases choisies parmi le groupe constitué par l'hydroxyde de sodium et l'hydroxyde de potassium.

4. Polysaccharide selon la revendication 1, capable de former des gels par réticulation avec des cations métalliques polyvalents choisis parmi le groupe constitué par Ti(IV), Al(III) ou Cr(III).

5. Solution de nettoyage caustique dans laquelle est incorporée une quantité épaississante efficace de biopolymer 35-80 tel que caractérisé à la revendication 1.

6. Solution de nettoyage acide dans laquelle est incorporée une quantité épaississante de biopolymer 35-80 tel que caractérisé à la revendication 1.

7. Procédé de préparation d'un hétéropolysaccharide désigné par l'appellation "biopolymer 35-80" contenant les sucres neutres de glucose et de galactose dans le rapport molaire approximatif de 7:1 ledit hétéropolysaccharide étant exempt de dérivés de l'acide uronique comprenant la mise en culture de l'espèce Agrobacterium ATCC 55341 dans un milieu aqueux par fermentation aérobie d'une source de carbone assimilable.

8. Micro-organisme ayant les caractéristiques d'identification de l'espèce Agrobacterium ATCC 55341.
